# EUROPEAN PATENT SPECIFICATION

(11) **EP 3 265 033 B1**
(45) Date of publication and mention of the grant of the patent: **04.12.2019**
(21) Application number: 16710584.0
(22) Date of filing: 04.03.2016
(51) Int. Cl.: A61F 2/46, A61B 17/15

(54) **BI-CRUCIATE KNEE SYSTEM**
DOPPELKREUZBAND-KNIESYSTEM
SYSTÈME DE PRÉSERVATION DE DEUX LIGAMENTS CROISÉS

(30) Priority: 05.03.2015 US 201514639522
(43) Date of publication of application: 10.01.2018
(73) Proprietor: Biomet Manufacturing Corp., Warsaw, IN 46582 (US)
(72) Inventor: METZGER, Robert, Wakarusa, Indiana 46573 (US); LOMBARDI, Adolph V., New Albany, Ohio 46054 (US); PETERS, Christopher, Murray, Utah 84107 (US); DECLAIRE, Jeffrey, Rochester Hills, Michigan 48307 (US); BEREND, Keith R., Columbus, Ohio 43215 (US); DELLA VALLE, Craig, Chicago, Illinois 60612 (US); DURCHOLZ, Bradley T., Warsaw, Indiana 46580 (US); BEDKE, Brice, North Manchester, Indiana 46962 (US)
(74) Representative: Mays, Julie
(86) International application number: PCT/US2016/020836
(87) International publication number: WO 2016/141274

(56) References cited:
- WO-A1-2010/029333
- WO-A1-2012/158604
- US-A1- 2004 167 531

## Description

### FIELD

The following disclosure relates generally to knee surgery and more specifically to an instrument set as defined in the claims.

Document WO 2010/029333 A1 discloses an exemplary instrument set including a tibial resection block and a stylus.

### SUMMARY

This section provides a general summary of the disclosure, and is not a comprehensive disclosure of its full scope or all of its features. According to a first aspect, there is an instrument set for preparing a proximal tibia during a bi-cruciate retaining procedure, according to claim 1. Preferred features are set out in the dependent claims.

An instrumentation set for preparing a proximal tibia during a bi-cruciate retaining procedure can include a tibial resection block and a stylus. The tibial resection block can be configured to be fixed to an anterior portion of the proximal tibia. The tibial resection block can define a slot that extends in a medial-lateral direction when the tibial resection block is fixed to the proximal tibia. The stylus can have a first block attachment feature and a second block attachment feature. The first block attachment feature can be offset from the stylus a first distance. The second block attachment feature can be offset from the stylus a second distance. The first and second block attachment features of the stylus can be selectively and alternatively received by the slot of the tibial resection block to position the stylus at distinct offset locations relative to the slot.

According to additional features, the first and second attachment features both comprise a lateral projection configured for receipt by the tibial resection block. The first and second block attachment features can oppositely extend from an attachment body. In other features, the instrument set can further include a vertical cut guide having a body, a medial arm and a lateral arm. The medial cut slot can be defined between the body and the medial arm. The lateral cut slot can be defined between the body and the lateral arm. The cut guide can further comprise a tongue extending therefrom. The tongue can be configured to be received by and slidably translate along the slot of the tibial resection block. A locking arm can be coupled to the cut guide and movable between an unlocked position and a locked position. In the unlocked position, the cut guide is permitted to translate relative to the tibial resection block. In the locked position, the locking arm engages the tibial resection block and inhibits movement of the cut guide relative to the tibial resection block. The tibial resection block can provide a seven degree posterior slot cut inclination.

According to other features, the instrument set can further comprise a tibial resection level guide having a handle end, an attachment portion and an engaging end. The attachment portion can be configured to be selectively received by the slot of the tibial resection block to selectively position the engaging end against a distal femoral resection surface. The instrument set can further include a tibial template tool having a first template end and a second template end. The first template end can have first inner fingers and first outer fingers. The second template end can have second inner fingers and second outer fingers. The first inner fingers can be spaced a first distance corresponding to a first tibial island. The second inner fingers can be spaced a second distance corresponding to a second tibial island. The first outer fingers can define a first outer tibial tray footprint. The second outer fingers can define a second outer tibial tray footprint.

An instrument set for preparing a proximal tibia during a bi-cruciate retaining procedure according to another example of the present disclosure can include a tibial resection block, a stylus, a vertical cut guide and a tibial resection level guide. The tibial resection block can be configured to be fixed to an anterior portion of the proximal tibia. The tibial resection block can define a slot that extends in a medial-lateral direction when the tibial resection block is fixed to the proximal tibia. The stylus can have a first block attachment feature that is offset from the stylus a first distance. The first block attachment feature of the stylus is selectively received by the slot of the tibial resection block to position the stylus at an offset location relative to the slot. The vertical cut guide can have a body, a medial arm and a lateral arm. A medial cut slot can be defined between the body and the medial arm. A lateral cut slot can be defined between the body and the lateral arm. The cut guide can further comprise a tongue that is configured to be received by and slidably translate along the slot of the tibial resection block. The tibial resection level guide can have a handle end, an attachment portion and an engaging end. The attachment portion can be configured to be selectively received by the slot of the tibial resection block to selectively position the engaging end against a distal femoral resection surface. All of the stylus, the vertical cut guide and the tibial resection level guide are selectively and alternatively received by the slot of the tibial resection block.

According to additional features the stylus further includes a second block attachment feature. The second block attachment feature can be offset from the stylus a second distance. The first and second block attachment features of the stylus are selectively and alternatively received by the slot of the tibial resection block to position the stylus at distinct offset locations relative to the slot. The first and second attachment features can both comprise a lateral projection configured for receipt by the tibial resection block. The first and second block attachment features can oppositely extend from an attachment body.

In other features, the vertical cut guide can further comprise a locking arm coupled to the cut guide and movable between an unlocked position and a locked position. In the unlocked position, the cut guide is permitted to translate relative to the tibial resection block. In the locked position, the locking arm engages the tibial resection block and inhibits movement of the cut guide relative to the tibial resection block. The instrument set can further include a tibial template tool having a first template end and a second template end. The first template end can have first inner fingers and first outer fingers. The second template end can have second fingers and second outer fingers. The first inner fingers are spaced a first distance corresponding to a first tibial island. The second inner fingers are spaced a second distance corresponding to a second tibial island. The first outer fingers can define a first outer tibial tray footprint. The second outer fingers can define a second outer tibial tray footprint.

A method for preparing a proximal tibia for receipt of a bi-cruciate implant incudes determining a resection level of the proximal tibia. A tibial cut block can be fixed relative to the proximal tibia based on the determination. The tibial cut block can have a slot defined thereon. A vertical cut guide can be located at the slot. The vertical cut guide can have a medial slot and a lateral slot. A vertical medial cut and a vertical lateral cut can be prepared into the proximal tibia while referencing the respective medial and lateral slots. The vertical cut guide can be removed from the slot. An attachment extending from a tibial resection level guide can be inserted into the slot. A depth of resection of the tibia can be verified with the tibial resection level guide.

According to one example paddles extending from the tibial resection guide can be engaged against a femoral surface to verify the depth of resection of the tibia. A preferred offset between a stylus and the tibial resection block can be determined. One of (i) a first block attachment feature provided on the stylus and (ii) a second block attachment feature provided on the stylus can be selected. The first block attachment feature can be offset a first distance from the stylus. The second block attachment feature can be offset a second distance from the stylus. The first and second distances can be distinct. The selected first or second block attachment feature can be mated to the slot of the tibial resection block. The vertical cut guide can be slidably translated along the slot defined in the tibial cut block until a desired medial-lateral position relative to the proximal tibia has been attained. The vertical cut guide can be fixed to the tibial cut block based on attaining the desired medial-lateral position. Fixing can include moving a locking arm extending from the vertical cut guide from an unlocked position to a locked position. In the locked position, a finger extending from the arm engages the tibial cut block.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is a perspective view of an exemplary 4-in-1 cutting block shown with a selectively attachable ACL protector.
FIG. 2 is a perspective view of the 4-in-1 block of FIG. 1.
FIG. 3 is an anterior view of an exemplary tibia shown prior to performing tibial preparation.
FIG. 4 is an anterior view of the tibia of FIG. 3 and shown subsequent to the tibial preparation.
FIG. 5 is an anterior perspective view of the tibia shown with an extramedullary tibial resection guide attached thereto.
FIG. 6 is an anterior perspective view of the proximal tibia and shown with a tibial resection block coupled to the extramedullary tibial resection guide and located against the proximal tibia.
FIG. 7 is a medial perspective view of the proximal tibia of FIG. 8 shown with a terminal end of a modular stylus engaged to the lowest point of the medial tibial plateau.
FIG. 8 is an anterior perspective view of the proximal tibia shown with the modular stylus positioned with a terminal end of the modular stylus engaged to the lowest point of the medial tibial plateau.
FIG. 9 is an anterior perspective view of the proximal tibia of FIG. 8 shown with the tibial resection block coupled with a modular stylus being adjusted to a desired location.
FIG. 10 is an anterior perspective view of the proximal tibia shown with a vertical cut guide coupled to the tibial resection block in line with an ACL and tibial island.
FIG. 11 is an anterior view of the tibia of FIG. 10 and shown with the vertical cut guide coupled to the tibial resection block in a locked position.
FIG. 12 is an anterior view of the proximal tibia shown subsequent to performing a pair of vertical cuts that will form lateral and medial sides of an ACL island made while referencing the vertical cut guide.
FIG. 12A is a cross-sectional view taken along lines 12A-12A of FIG. 12.
FIG. 12B is a perspective view of a vertical cut guide constructed in accordance to additional features.
FIG. 12C is a cross-sectional view of the vertical cut guide of FIG. 12B
FIG. 13 is a superior view of the proximal tibia shown with a pre-trial spacer located atop of the lateral plateau to verify the height of tibial bone that was resected.
FIG. 14 is an anterior perspective view of the proximal tibia and pre-trial spacer shown in FIG. 13.
FIG. 15 is a superior view of the proximal tibia shown with a Rongeur tool initially located for resection of the anterior portion of the tibia.
FIG. 16 is a close-up view of the anterior portion of the ACL island of FIG. 15.
FIG. 17 is an anterior perspective view of the proximal tibia of FIG. 15.
FIG. 18 is an anterior perspective view of the tibia of FIG. 17 and shown subsequent to resection of the anterior island and using a rasp to clean up the surface surrounding the ACL island.
FIG. 19 is an anterior perspective view of the proximal tibia shown with a tibial plateau angle gage disposed thereon.
FIG. 20 is a close-up view of a scale of the tibial plateau angle gage of FIG. 19.
FIG. 21 is a perspective view of a spacer tool used to verify a medial and lateral gap.
FIG. 22 is a superior view of the proximal tibia shown using an optional anterior/posterior sizer to verify tibia size.
FIG. 23 is a close-up view of a scale of the sizer shown in FIG. 22.
FIG. 24 is a perspective view of the proximal tibia and shown with a tibial template and anterior/posterior sizer disposed thereon used to verify size, rotation and slope.
FIG. 25 is a lateral view of the proximal tibia shown with the tibial template and anterior/posterior sizer of FIG. 24 disposed thereon.
FIG. 26 is an anterior perspective view of the proximal tibia and shown with the tibial template placed thereon and shown with a drill aligned for receipt by a medial anterior grill guide on the tibial template.
FIG. 27 is an exploded front perspective view of a tibial mask and tibial template.
FIG. 28 is an anterior perspective view of the proximal tibia and shown with a toothbrush keel blade aligned for receipt into a medial passage provided in the tibial template.
FIG. 29 is an anterior view of the proximal tibia of FIG. 28 and shown with the toothbrush keel blade received by the medial passage of the tibial template during formation of a medial groove in the tibia.
FIG. 30 is a front perspective view of a tibial tray trial and tibial tray trial insert constructed in accordance to one example of the present teachings.
FIG. 31 is a front perspective view of the tibial tray trial and tibial tray trial insert shown in an assembled position.
FIG. 32 is an anterior perspective view of the prepared proximal tibia shown with the tibial tray trial and tibial tray trial insert located thereon.
FIG. 33 is a medial perspective view of the proximal tibia and shown with a tibia bearing trial handle and tibial impactor coupled to the tibial tray trial.
FIG. 34 is an anterior perspective view of the proximal tibia of FIG. 33 and bearing trial handle tool.
FIG. 35 is an anterior perspective view of the proximal tibia of FIG. 34 shown with the bearing trial handle tool positioning a bearing onto the tibial tray.
FIG. 36 is a front perspective view of the proximal tibial of FIG. 35 shown with a medial and lateral bearing coupled to the tibial tray.
FIG. 37 is a front perspective view of the tibial tray of FIG. 36 shown with a femoral trial used to check range of motion.
FIG. 38A is a front perspective view of a mask having a locking feature according to other features of the present disclosure;
FIG. 38B illustrates the mask of FIG. 38A shown coupled to a tibial template according to one example of the present disclosure;
FIG. 39 is a front perspective view of a tibial sizer according to one example of the present disclosure;
FIG. 40 is a front perspective view of a spacer block constructed in accordance to one example of the present teachings;
FIG. 41 is a front perspective view of another spacer block constructed in accordance to another example of the present teachings;
FIG. 42 is a front perspective view of a presetter tool constructed in accordance to one example of the present disclosure;
FIG. 43 is a front perspective view of an alignment guide constructed in accordance to one example of the present disclosure;
FIG. 44 is a front perspective view of a rasp constructed in accordance to one example of the present disclosure;
FIG. 45A is a front perspective view of a stylus constructed in accordance to one example of the present disclosure;
FIG. 45B is a front perspective view of the stylus of FIG. 45A shown with a first attachment portion received by the slot of the tibial resection block;
FIG. 45C is a front perspective view of the stylus of FIG. 45A shown with a second attachment portion received by the slot of the tibial resection block;
FIG. 46 is a front view of a tibial resection cut block constructed in accordance to one example of the present disclosure;
FIG. 47 is a front perspective view of an impactor constructed in accordance to one example of the present disclosure;
FIG. 48A is a front perspective view of a tibial resection level guide constructed in accordance to one example of the present disclosure;
FIG. 48B is a front perspective view of the tibial resection level guide of FIG. 48A shown mated with the slot of the tibial resection guide and having an engaging end engaged to distal femoral resection surface;
FIG. 49 is a front perspective view of a double rasp tool constructed in accordance to one example of the present disclosure;
FIG. 50 is a front perspective view of a tibial resection block constructed in accordance to one example of the present disclosure;
FIG. 51 is a front perspective view of an indicator tool constructed in accordance to one example of the present disclosure;
FIG. 52 is a front perspective view of a tibial template tool constructed in accordance to one example of the present disclosure;
FIG. 53 is a front perspective view of a holding tool constructed in accordance to one example of the present disclosure;
FIG. 54 is a front perspective view of a tibial recutting guide constructed in accordance to one example of the present disclosure;
FIG. 55 is a top view of an offset rasp tool constructed in accordance to one example of the present disclosure;
FIG. 56 is a top perspective view of an exemplary tibial tray constructed in accordance to one example of the present disclosure; and
FIG. 57 is a side view of an exemplary bearing constructed in accordance to one example of the present disclosure.

Corresponding reference numerals indicate corresponding parts throughout the several views of the drawings.

### DETAILED DESCRIPTION

The following description will focus on preparation of a left knee for receipt of a bi-cruciate knee implant. In this regard, the following description will be directed toward various methods and techniques using instrumentation for preparing a left knee using a bi-cruciate knee system. It will be appreciated however, that the same may be adapted for use with a right knee.

While the intended focus of the instant application will be directed specifically to preparation of the tibia and related implants, a brief description of an exemplary preparation of a left femur will be described. In order to assess bone stock, potential ligament instability and the anatomical axis, a standing anterior/posterior x-ray may be used. In some examples, a 91.44 cm (36 inch) long standing anterior/posterior x-ray may be used. Initially, the angle between the anatomic and mechanical axis may be determined while assuring that the distal femoral cut is perpendicular to the mechanical axis. At this time, the femoral component size may be estimated pre-operatively by using lateral view x-rays and radio graphic templates. The appropriate size femoral component may be confirmed intraoperatively.

An intramedullary (IM) drill may be used to penetrate the intracondylar notch and dense cancellous bone of the distal femur to a depth of approximately 3.5 - 5 cm (1.5 - 2 inches) A 0.95 cm (0.375 inch) drill may be used to penetrate the distal femur. The canal entry location may be placed one centimeter above the insertion of the posterior cruciate ligament and slightly medial in the intracondylar notch. The appropriate left or right valgus wing may be chosen and slid onto the IM rod. The IM rod may be introduced into the femoral canal to depressurize the canal. The valgus wing may be slid until it rests against the medial distal condyle. The Slidex® Distal Resection Block and cut block adapter are both slid into the anterior holes of the valgus wing until the Slidex® Distal Resection Block contacts the anterior cortex of the femur.

To confirm the valgus angle, the alignment handle can be inserted into the cut block adapter and a 0.64 cm (1/4 inch) alignment rod can be inserted and extended to the center of the femoral head. The Slidex® Distal Resection Block can then be pinned into place using 0-32 cm (1/8 inch) quick release drill pins in the most proximal pin holes of the block. The valgus wing can then be removed by removing the IM rod and pulling the valgus wing and cut block adapter distally away from the distal resection block leaving the Slidex® Distal Resection Block in place. Two resection slots of 0 or +3 mm are available for the distal resection. The 0 mm slot will resect 9 mm from the most prominent part of the medial distal condyle. If additional distal resection is required, the +3 mm slot will resect 12 mm. If additional distal resection is required beyond the +3 mm slot, the resection guide can be shifted proximally by utilizing the +2 or +4 mm 0.32 cm (1/8 inch) pin holes. A 0.14 cm (0.054 inch) saw blade can be used to complete the distal resection through the selected slot. The resected distal femur can be checked by using a flat instrument. The bone surface may be re-cut or filed as necessary to ensure proper resection. For additional stability, the femoral block handle can be utilized.

An exemplary method of femoral sizing will now be described. Initially, the adjustable anterior/posterior sizer may be placed against the resected distal surface with the feet in contact with the posterior condyles of the femur. In a first option, fixed rotation feet may be used. In another option, adjustable rotation feet may be used. An adjustable dial can be used with the anterior/posterior sizer. The adjustable rotation feet are available in left and right varieties with the ability to set an external rotation from 0 to 10 degrees. In one example, it is recommended that an initial setting of 3 degrees of rotation be utilized. The femoral component size can now be read from the central scale. If the size indicated is in between standard sizing or a larger flexion gap is desired, a choice may be made to choose the smaller size and shift the femoral 4-in-1 block placement anteriorly. In order to shift the component anteriorly, a screw mechanism in the central portion of the sizer is turned which raises the level of drill holes in one millimeter increments. A scale is located on the sizer to indicate how far the component will be anteriorly shifted. If medial/lateral width is a concern, the appropriately sized medial/lateral width checker can be inserted into the anterior/posterior sizer to further evaluate the proper size of the femur. Next, two 4-in-1 cutting block location holes are drilled utilizing a 0.32 cm (1/8 inch) drill pin. In one example, the final medial/lateral position of the femoral component is not determined during this step, but is addressed later in the technique.

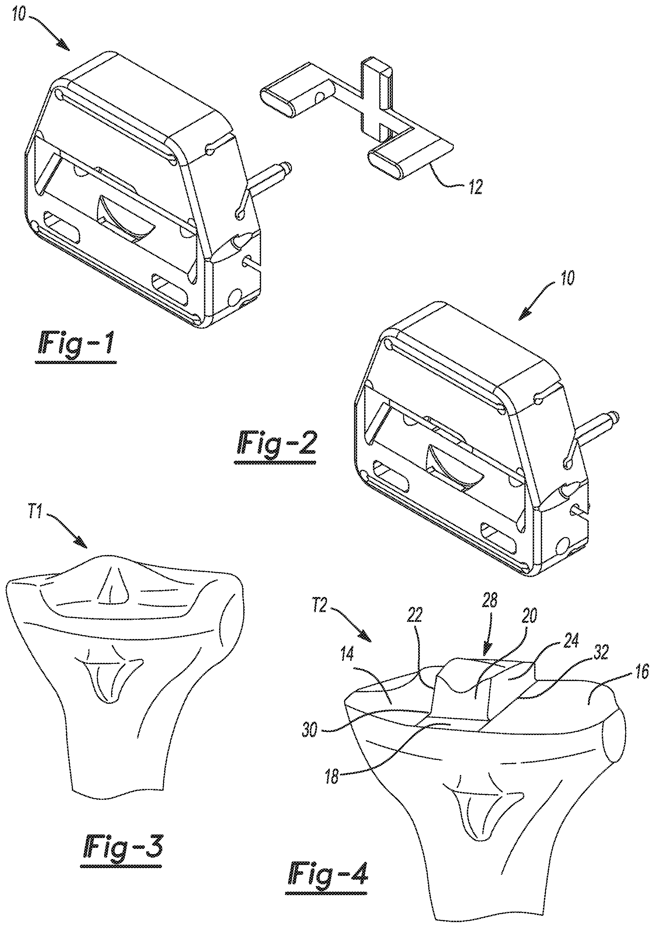

With initial reference now to FIGS. 1 and 2, initial preparation of the distal femur using a 4-in-1 block 10 according to the present teachings will be described. At the outset, a surgeon may choose the desired 4-in-1 block 10 that matches the selected size on the anterior/posterior sizer and place it into the 0.32 cm (1/8 inch) holes drilled into the distal femur. A 0.14 cm (0.054 inch) feeler blade can be used to determine the amount of anterior bone resection. If the feeler blade indicates a probability of notching, an anterior/posterior femoral shift block may be used to adjust the cut block holes anteriorly in one millimeter increments. Notably, moving the block anteriorly will resect additional posterior condylar bone 0.32 cm (1/8 inch) pins can be placed in the side holes provided on the femoral 4-in-1 block 10. The anterior/posterior block must be sitting flush against the distal femur at this point. An ACL protector 12 may be secured into place relative to the 4-in-1 block 10. The ACL protector 12 can be used to block the blade from inadvertently cutting the ACL. Once the position of the 4-in-1 block 10 is satisfactory, a surgeon can resect the anterior and posterior bone, and the anterior and posterior chamfers using a 0.14 cm (0.054 inch) saw blade. Again, care must be taken not to cut the ACL while making the posterior and posterior chamfer boney resections.

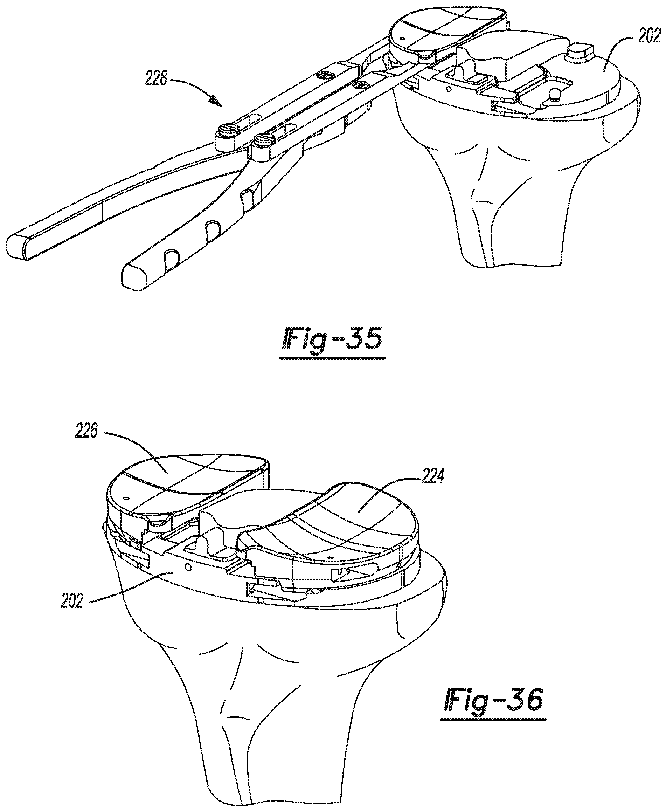

With reference now to FIGS. 3-37, preparation of a proximal tibia for a bi-cruciate knee system according to a first example will be described. FIG. 3 illustrates a tibia T1 prior to performing the instant surgical technique. FIG. 4 illustrates a tibia T2 subsequent to performing the tibial technique according to the present teachings. Of note, the tibia T2 includes a medial plateau 14, lateral plateau 16, anterior plateau 18, anterior chamfer wall 20, medial vertical wall 22, and lateral vertical wall 24. The anterior chamfer wall 20, the medial vertical wall 22, and the lateral vertical wall 24 can collectively cooperate to form an ACL island 28. A radius 30 is formed at a transition between the medial plateau 14 and the medial vertical wall 22. Similarly, a radius 32 is formed at a transition between the lateral plateau 16 and the lateral vertical wall 24.

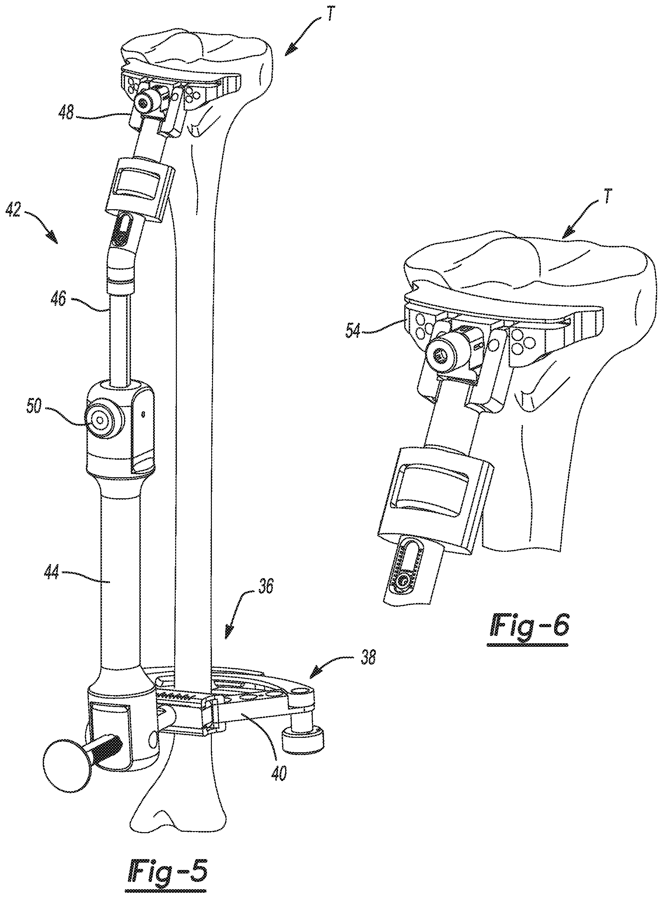

With reference now to FIGS. 5-29, resection of the tibia T will be described. With the knee flexed, spring loaded arms 36 and 38 of an ankle clamp 40 are located around the distal tibia T just around the malleoli. The ankle clamp 40 can generally be attached to an extramedullary tibial resection guide 42. The extramedullary tibial resection guide 42 can further comprise a handle portion 44, a telescoping rod portion 46, and a resection block connecting portion 48. A button 50 can be provided on the extramedullary tibial resection guide 42 that can control telescoping action of the rod portion 46 generally from the handle portion 44.

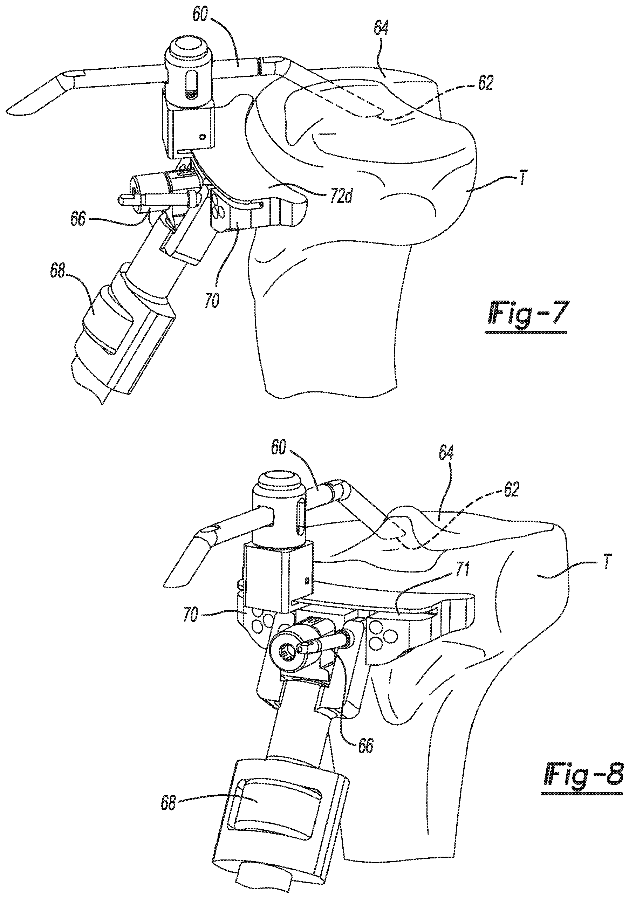

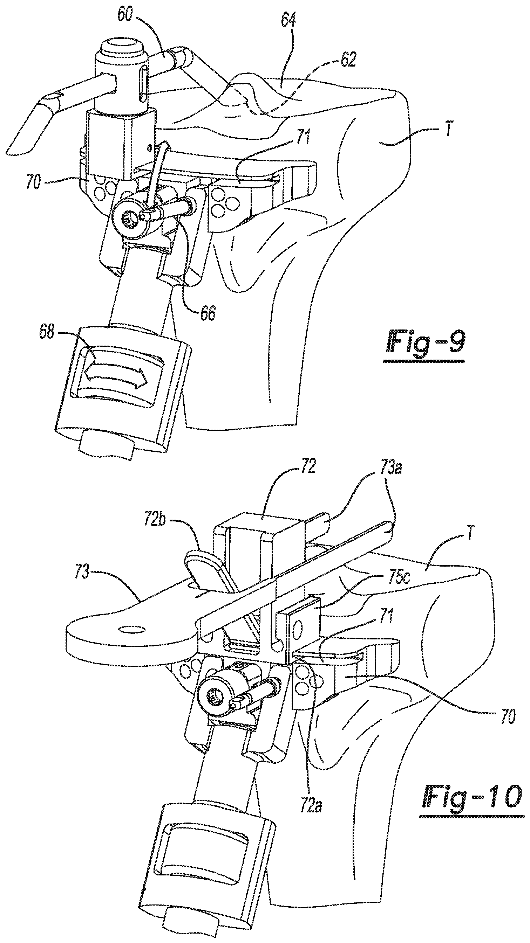

At this point, a tibial resection block 54 (FIG. 6) can be placed against the proximal tibia T. Returning now to FIG. 5, from the sagittal view, the side of the extramedullary tibial resection guide 42 is adjusted such that it is generally parallel with the shaft of the tibia T. The tibial resection block is set at 4 degrees of slope (other measurements may be used) when attached to the extramedullary guide. Once adjustment of the resector axis is correct in the medial/lateral view, the resection block connecting portion 48 is rotated until the shaft of the resector is just medial to the tibial tubercle. Using a stylus 60 (FIGS. 7 and 8), the extramedullary tibial resection guide 42 is adjusted such that a terminal end 62 of the stylus 60 is engaged to a lowest point of the medial tibial plateau 64. Using a 0.32 cm (1/8 inch) pin 66, the extramedullary tibial resection guide 42 is secured to the tibia T. A dial 68 may be used to fine tune the resection level prior to making any cut (FIG. 9).

Of note, the stylus 60 is set for a 4 mm resection. Prior to pinning the extramedullary tibial resection guide 42 in place, make sure to allow for adjustability of the height of a tibial resection cut block 70. The tibial resection cut block 70 can define a horizontal slot 71. Once the resection level is set, the stylus 60 can be removed. A vertical cut guide 72 can then be attached to the tibial resection cut block 70 (FIG. 10).

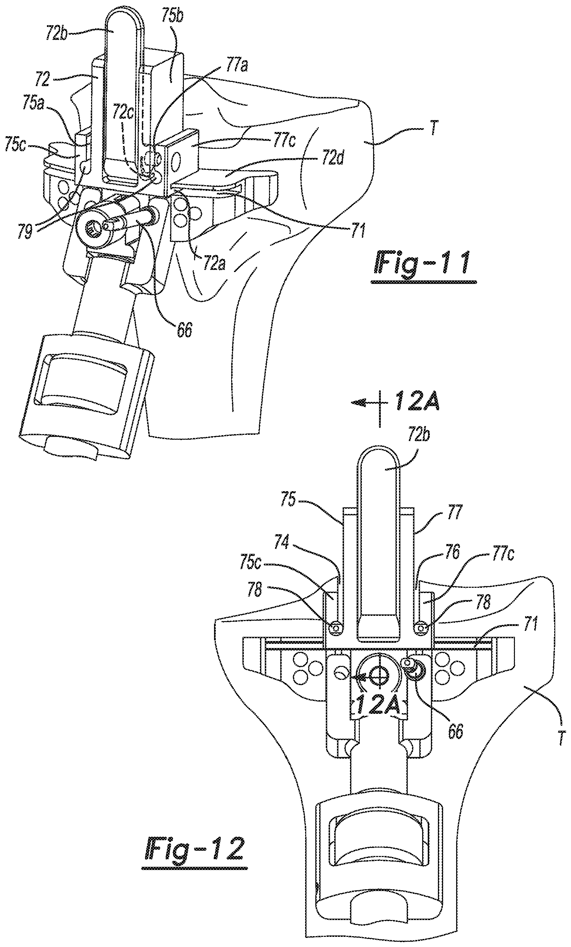

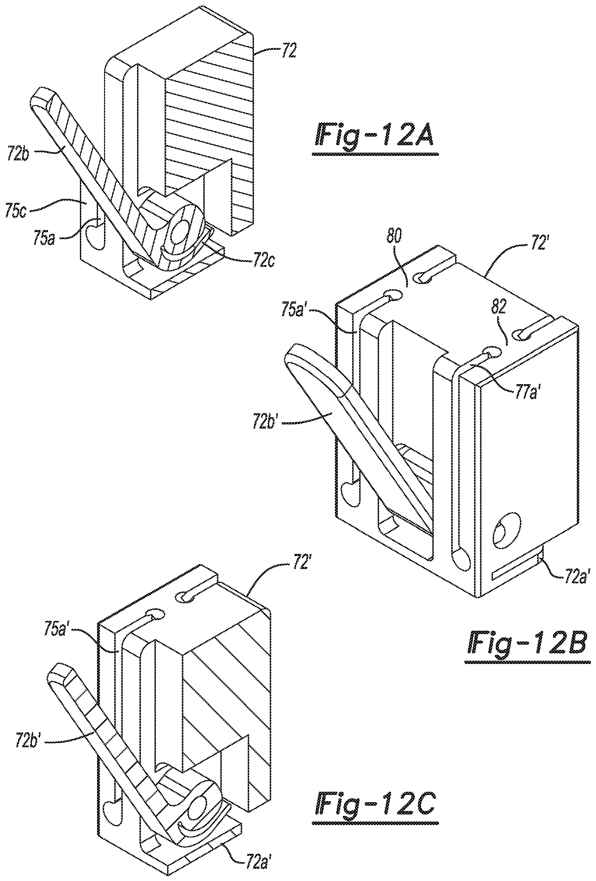

The vertical cut guide 72 can then be adjusted to an appropriate position (in a medial/lateral direction along the slot 71) to make the desired vertical cuts. Specifically, a tongue 72a extending from the vertical cut guide 72 can slide along the slot 71. An alignment guide 73 can be used to aid in the positioning of the vertical cut guide 72. The alignment guide 73 generally includes a pair of parallel and elongated arms 73a that slidably locate on opposite sides of the vertical cut guide 72. Of note, the vertical cuts will determine the final tibial component rotation. It is important to leave equal amounts of bone on the medial and lateral aspect of the ACL fibers. At this point, the vertical cut guide 72 can be clamped in place by rotating a locking arm 72b from an unlocked position shown in FIG. 10 to a locked position shown in FIGS. 11 and 12. In one example, the locking arm 72b can have a finger 72c that rotates into fixed engagement with an upper surface 72d of the cut block 70. With a reciprocating saw, a vertical medial cut 74 can be prepared while passing a saw through a medial slot 75a defined between a main body 75b of the vertical cut guide 72 and a medial arm 75c. The vertical medial cut 74 may be prepared while referencing a medial surface 75 of the vertical cut guide 72. It will be appreciated that the vertical medial cut 74 may be prepared while concurrently referencing the medial arm 75c. After the vertical medial cut 74 has been prepared, the vertical lateral cut may be made. The vertical lateral cut 76 can be prepared while passing a saw through a lateral slot 77a defined between the main body 75b of the vertical cut guide 72 and a lateral arm 77c. The vertical lateral cut 76 may be prepared while referencing a lateral surface 77 of the vertical cut guide 72. It will be appreciated that the vertical lateral cut 76 may be prepared while concurrently referencing the lateral arm 77c, Headless vertical pins 78 can be located through partial bores 79 (FIGS. 11 and 12) provided in the vertical cut guide 72 driven into the anterior tibia T. The vertical medial cut 74 and the vertical lateral cut 76 can both be prepared using a saw blade having teeth or cutting structure consistent for forming the radius cuts 30 and 32 identified in FIG. 4. Notably, by incorporating a radius at this transition, the bone at the transition between the respective medial and lateral plateaus 14, 16 and ACL island 28 (FIG. 4) can be stronger as compared to a transverse, 90 degree intersecting cut. Next, the vertical cut guide 72 is removed from the headless vertical pins 78. The medial side of the tibia T may then be horizontally resected.

With reference to FIG. 12A, a cross-sectional view of the cut guide 72 is shown. FIGS. 12B and 12C show an alternate vertical cut guide 72'. Unless otherwise described herein, the cut guide 72' incorporates similar features as the cut guide 72 that are identified with like reference numerals having a prime suffix. The cut guide 72' provides a captured vertical medial slot 75a' and a captured vertical lateral slot 77a'. Specifically, an upper medial wall 80 and an upper lateral wall 82 close the respective vertical medial slot 75a' and the vertical lateral slot 77a'. The upper medial and lateral walls 80 and 82 can assist in maintaining a saw blade within the respective medial and lateral slots 75a' and 77a'.

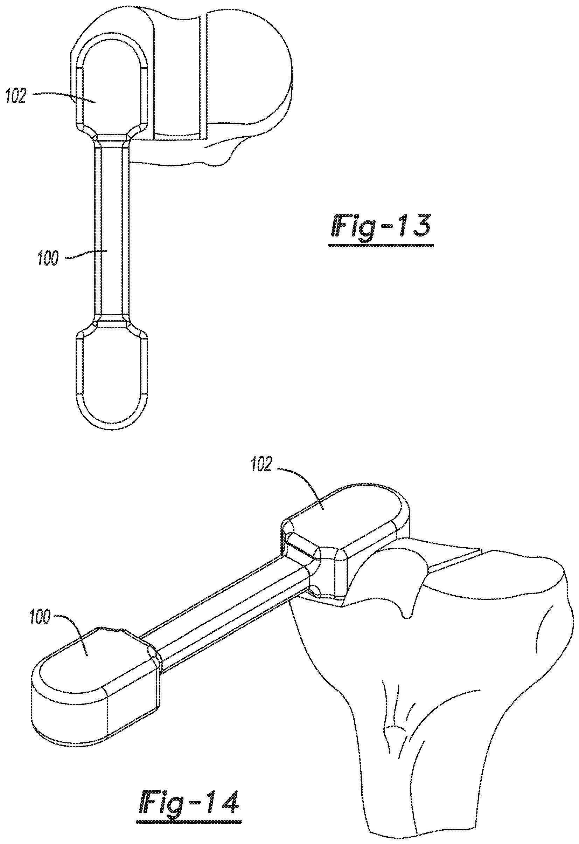

At this point, the medial side gap may be verified in extension using an 8/9 mm spacer block 100 (FIGS. 13-14). If the 9 mm spacer portion 102 is too tight, additional tibial bone will need to be removed. This can be done by simply dialing the resection block down 1 mm. Once the medial side extension gap is adequate, the lateral side of the tibia T is horizontally resected with the headless vertical pins 78 left in place. The headless vertical pins 78 protect against undercutting the ACL island 28.

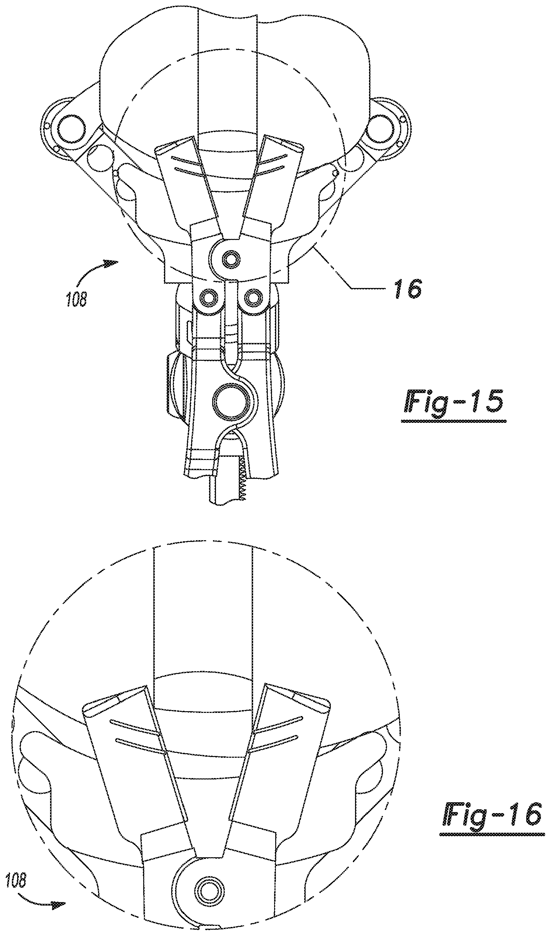

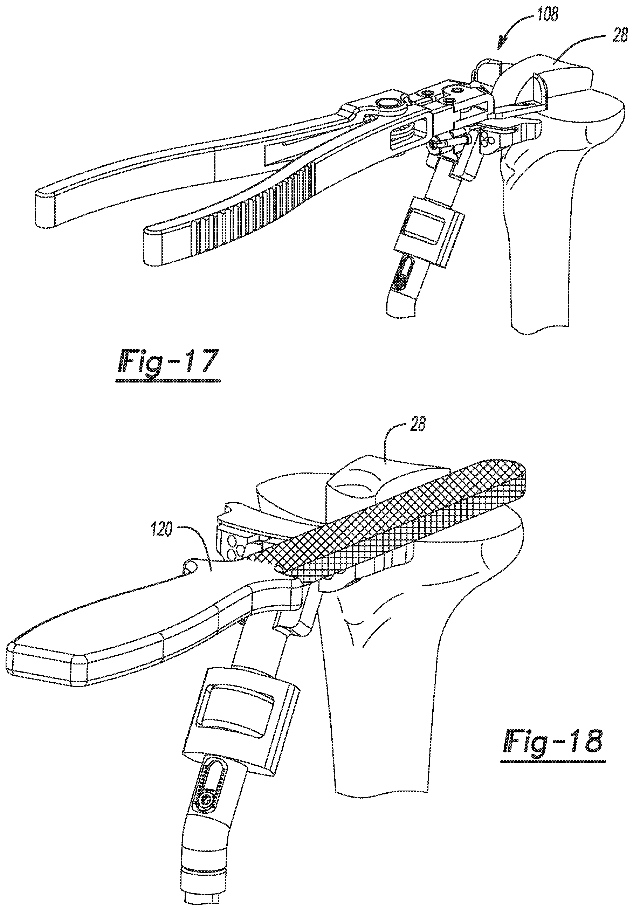

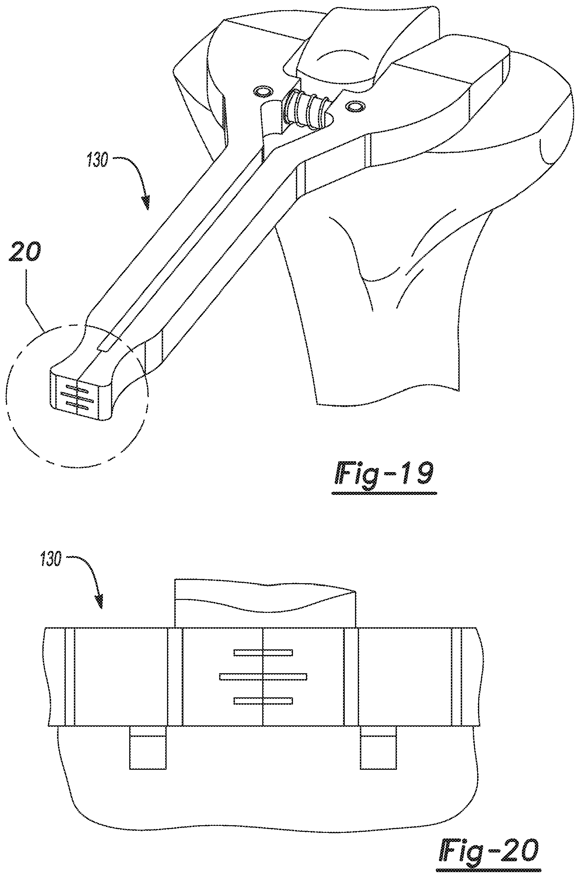

As illustrated in FIGS. 15-17, a Rongeur tool 108 can be used to remove the anterior bone making sure to round the corners of the anterior island. Next, an ACL island rasp 120 (FIG. 18) is used to clean the resected tibia T to ensure that there are no rough edges around the ACL island 28 and respective medial and lateral plateaus 14 and 16. Using the tibial plateau angle gage 130 (FIG. 19), the tibial slope cuts are verified to have an equal amount of slope. This will be important for the tibial base plate to be secured properly, and for the proper wear and function of the system.

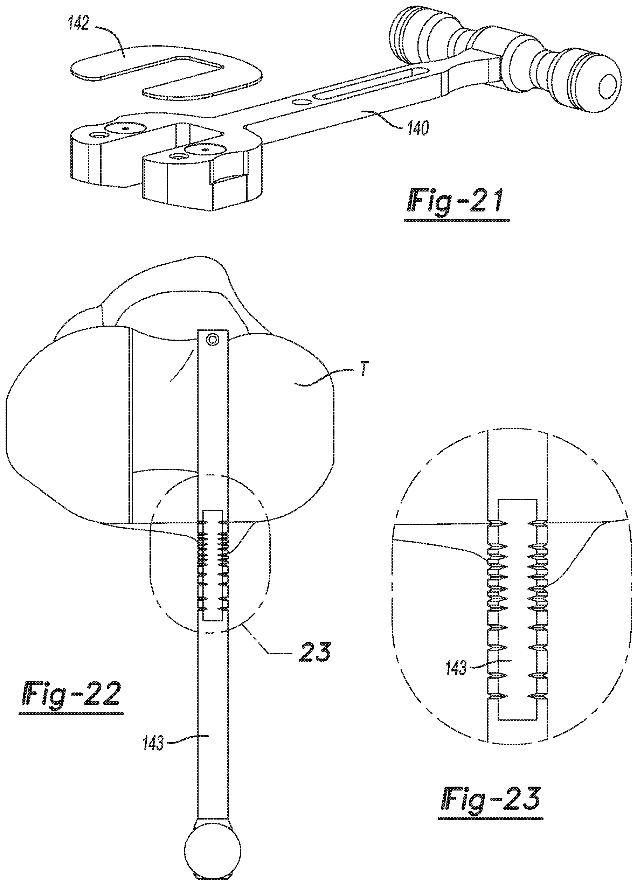

Turning now to FIG. 21, tibial sizing for an intact and functional ACL will be described. The medial and lateral gaps are verified using a spacer tool 140. A series of 1 mm spacers 142 may be magnetically coupled as needed. Rotation and slope may also be verified. Optionally, the tibia T may be sized with an anterior/posterior sizer 143 (FIGS. 22 and 23).

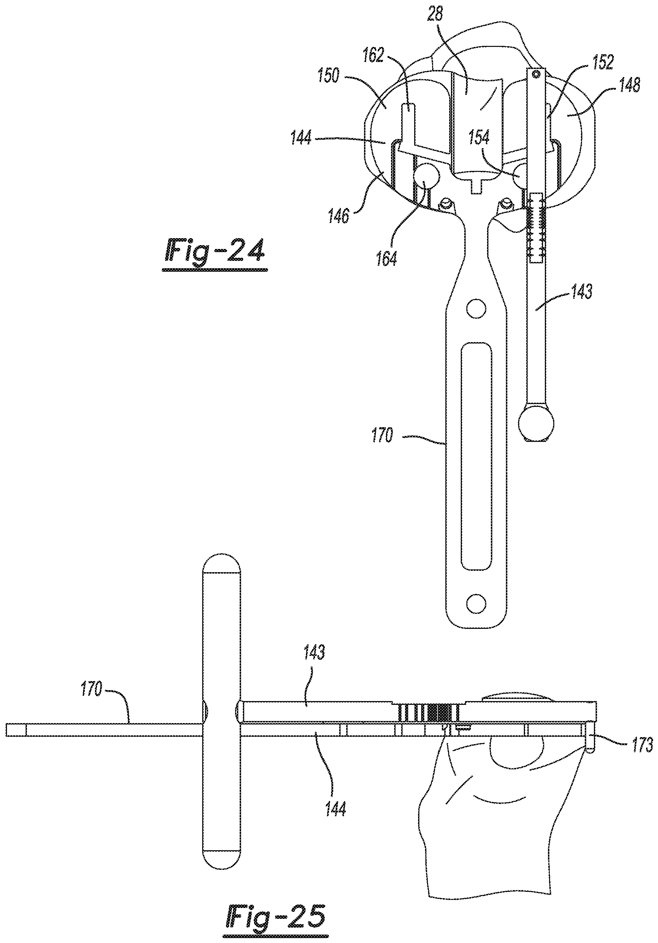

The tibia T may then be sized with a tibial template 144 (FIGS. 24-25). The tibial template 144 generally comprises a U-shaped body portion 146 having a lateral side 148, and a medial side 150. A lateral passage 152 and a lateral anterior drill guide 154 can be provided on the lateral side 148. Similarly, a medial passage 162 and medial anterior drill guide 164 can be provided on the medial side 150. Because rotation is determined by the position of the ACL island 28, it is important to check for accurate rotation. Base rotation can be made relative to the tibial tubercle and the malleolar axis. At this point, an extramedullary alignment check can be made by placing a 0.64 cm (1/4 inch) alignment rod through a handle 170 of the tibial template 144. Slight external rotation is preferred to optimize patellofemoral tracking. Once the final rotation has been determined, the position can be marked by extending anterior marks of the tibial template 144 onto the anterior tibia such as by electrocautery. A locator pin 173 extending from the anterior/posterior sizer 143 can be located around the posterior edge of the tibia T. Extra caution should be used to avoid internal rotation of the tibial template 144 due to the presence of lateral soft tissue.

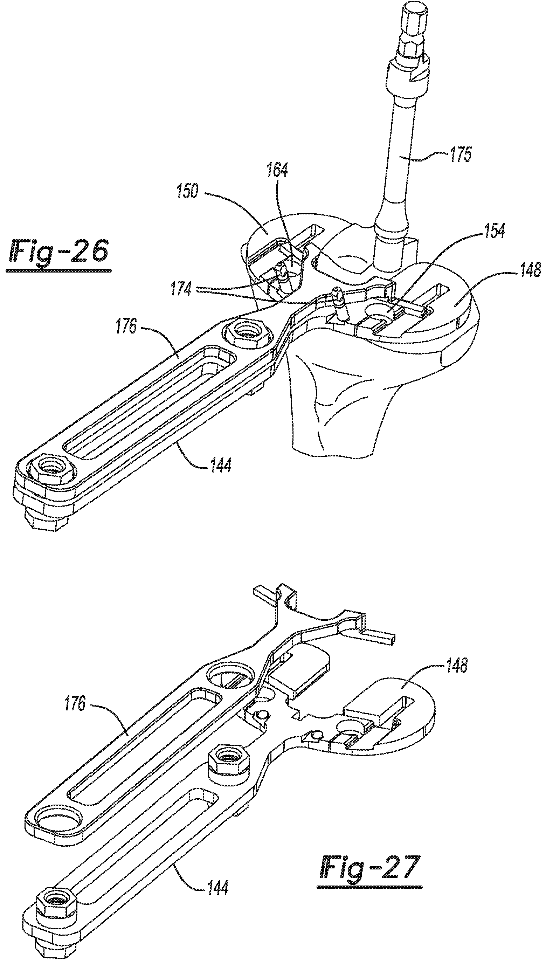

Tibial preparation for an intact and functional ACL will now be described. With the tibial template 144 in proper position (FIG. 26), such as by way of pins 174, a drill 175 can be used to prepare an anterior hole while referencing the lateral anterior drill guide 154. A tibial mask 176 may be coupled to the tibial template 144. In one example, a 0.32 cm (1/8 inch) drill 175 may be used (FIG. 26). Next, another anterior hole can be drilled with the drill 175 while referencing the medial anterior drill guide 164.

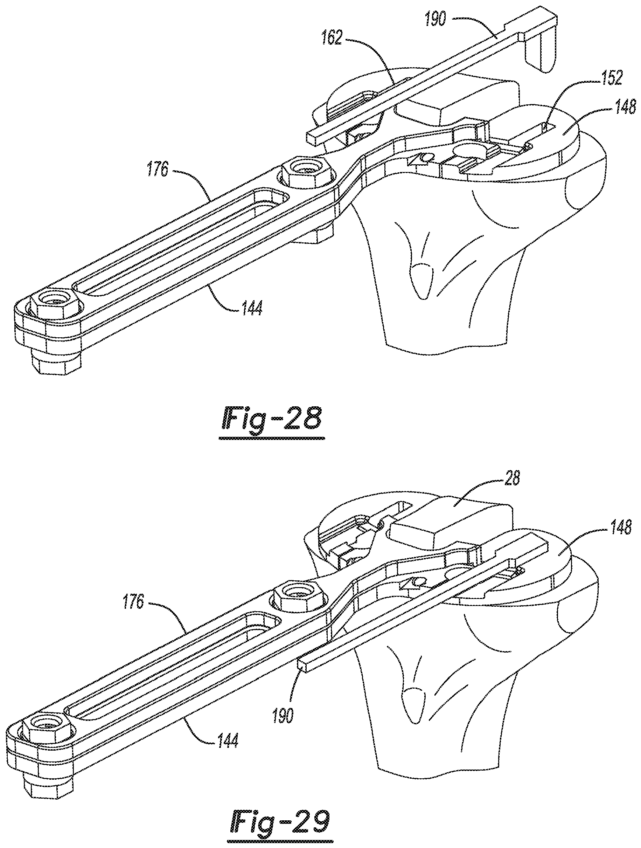

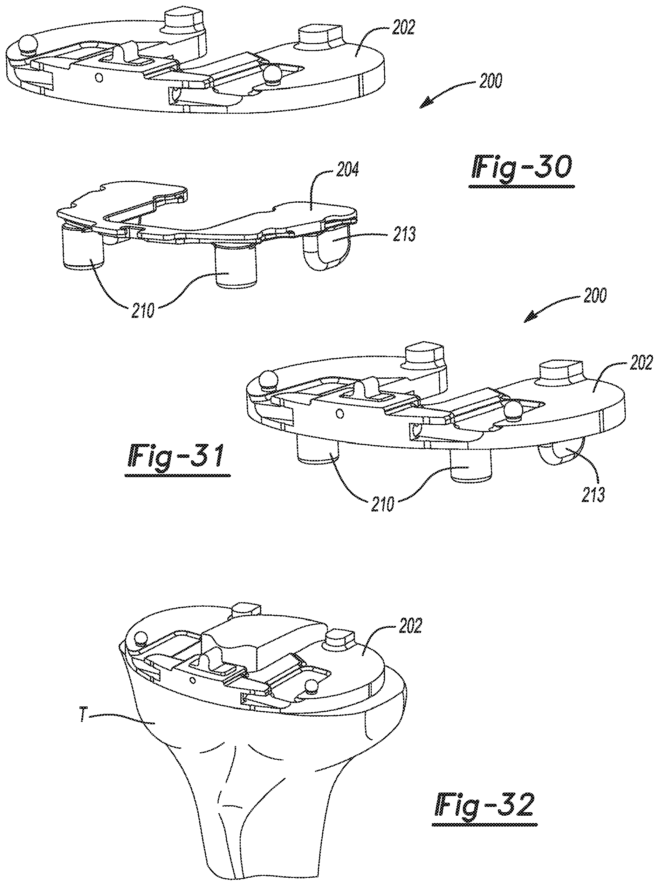

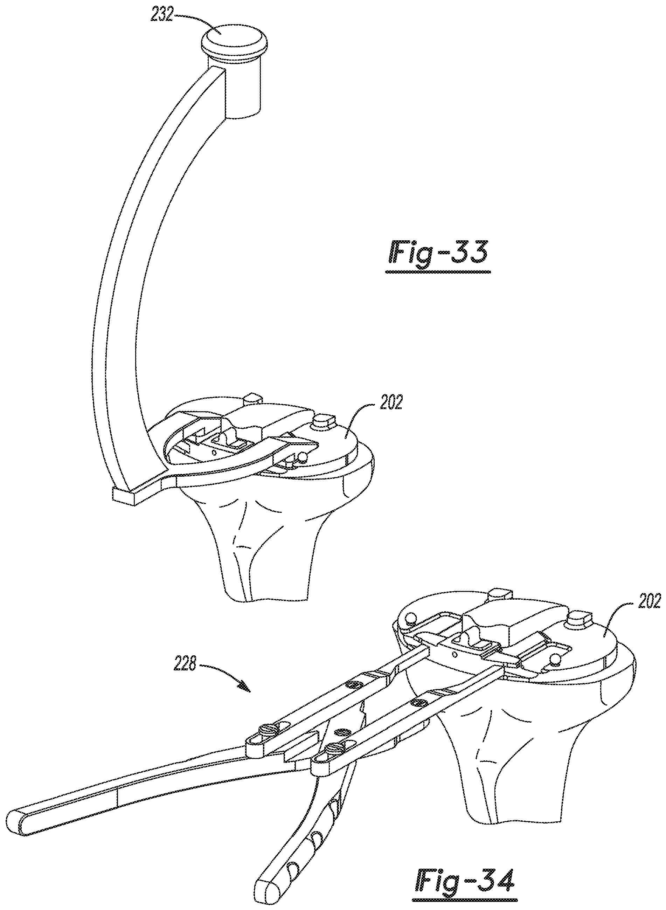

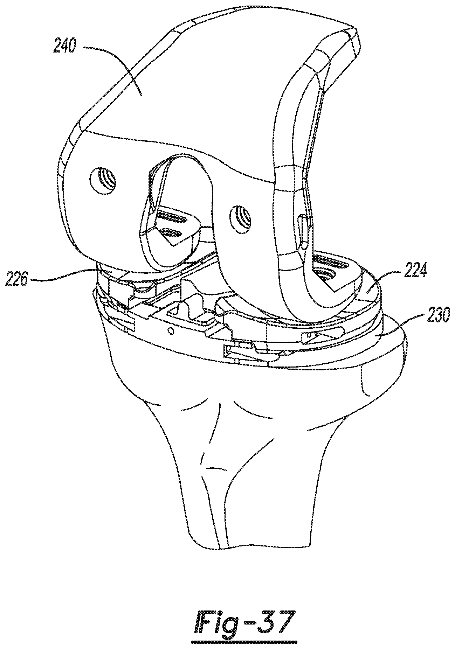

With the tibial template 144 secured in place, a toothbrush keel blade 190 can be used to prepare both the medial and lateral tibia for the keeled base plate. Specifically, the toothbrush keel blade 190 can be inserted through the lateral passage 152 and the medial passage 162 (FIGS. 28 and 29). While the tibia T is being prepared, the tibial trial assembly 200 (FIGS. 30 and 31) can be prepared. The tibial trial assembly 200 can include a tibial tray trial 202 and tibial tray trial insert 204. Once tibial preparation is complete, the tibial template 144 can be removed from the proximal tibia. The tibial tray trial 202 can have multiple versions that provide various dimensions. Similarly, the tibial tray trial insert 204 can also provide various dimensions suitable for the needs of a particular patient. Of note, the tibial tray trial insert 204 includes pegs 210 and keels 213. The pegs 210 have a spacing that corresponds to the passages made earlier with the drill 175. Similarly, the keels 213 have dimensions suitable for insertion into the grooves prepared with the toothbrush keel blade 190. As illustrated in FIG. 33, a tibial tray trial 202 is shown being impacted onto the tibia T using a tibial impactor 232. As illustrated in FIGS. 34-36, a lateral tibial bearing trial 224 and a medial tibial bearing trial 226 can be coupled to the tibial tray trial 202 using a bearing trial handle tool 228 and trialed. Also, the tibial tray trial 202 can be positioned with the bearing trial handle tool 228 (FIG. 34). As shown in FIG. 37, a femoral trial 240 can be used to verify range of motion.

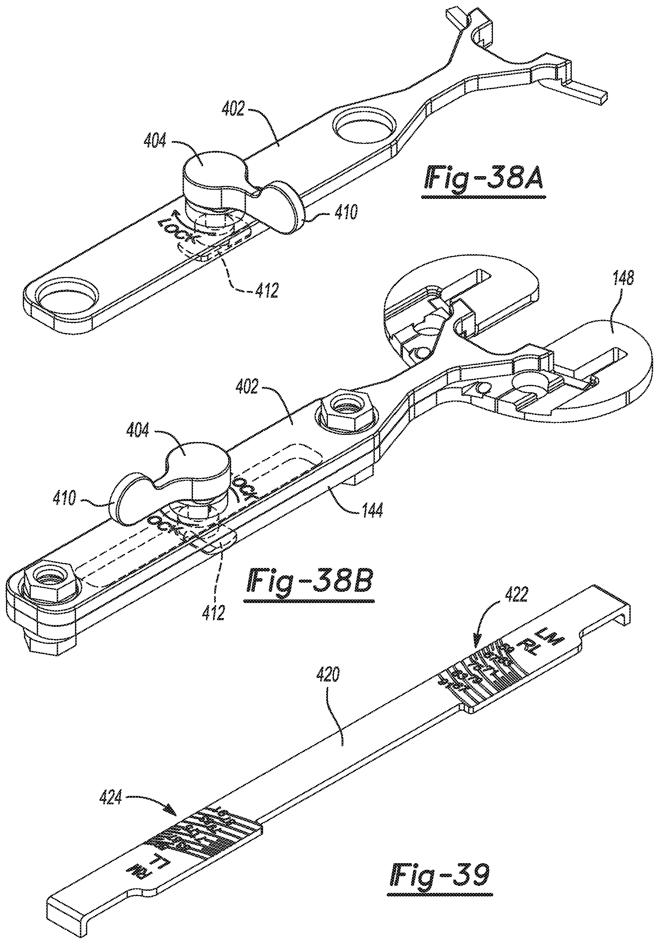

With reference now to Figures 38A-55, instruments configured to prepare the proximal tibia according to additional features will be described. FIG. 38A illustrates a mask 402. The mask 402 includes a locking feature 404 having an actuating lever 410 and a catch 412. The actuating lever 410 can be rotated from an unlocked position (FIG. 38A) to a locked position (FIG. 38B) to lock the mask 402 to the tibial template 144. In one configuration, the catch 412 can be advanced through a slot 414 defined on the tibial template 144. Rotation of the actuating lever 410 (from the unlocked position shown in FIG. 38A) can cause the catch 412 to locate under an arm of the tibial template 144 and lock the mask 402 to the tibial template 144 (FIG. 38B).

FIG. 39 illustrates a tibial sizer 420. The tibial sizer 420 can be used to size a tibia in the anterior/posterior direction (see also FIG. 22). The tibial sizer 420 is formed of flat or planar material for ease of positioning. The tibial sizer includes first indicia 422 and second indicia 424. The first indicia 422 can correspond to left medial and right lateral measurements. The second indicia 424 can correspond to right medial and left lateral measurements.

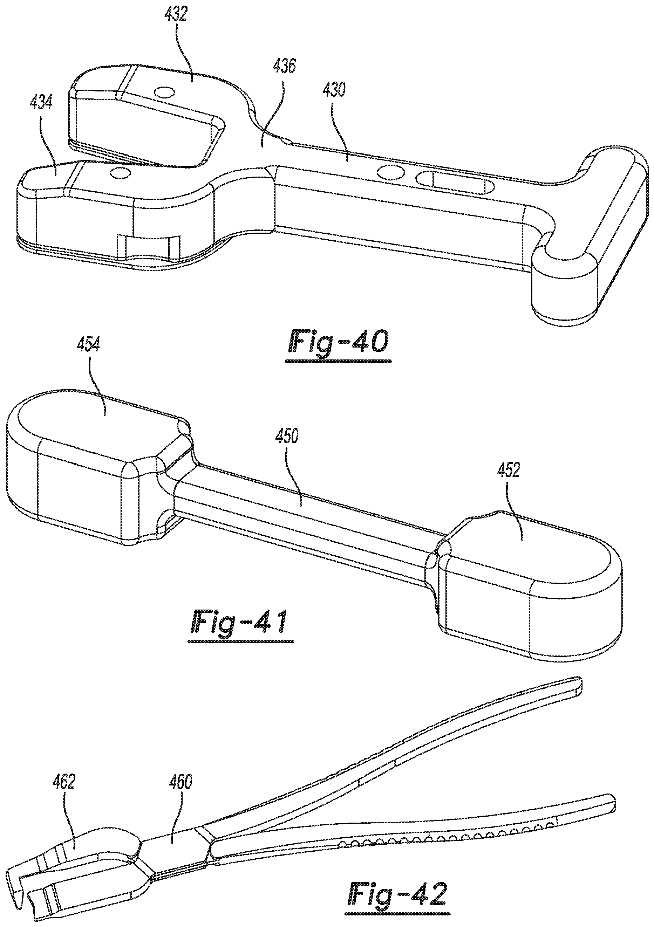

FIG. 40 illustrates a spacer block 430. The spacer block 430 includes fingers 432 and 434 extending from a central portion 436. The fingers 432 and 434 can diverge from the central portion 436 such that they are open to inhibit impingement on the ACL island 28. In one example, the spacer block 430 is formed of plastic. The fingers 432 and 434 can be 9mm thick. Other thicknesses are contemplated. A spacer block 450 is shown in FIG. 41. The spacer block 450 can include a first spacer block portion 452 and a second spacer block portion 454. The first spacer block portion 452 can be 9mm. The second spacer block portion 454 can be 10mm. The spacer block 450 can be used to verify the medial and lateral side gaps in extension.

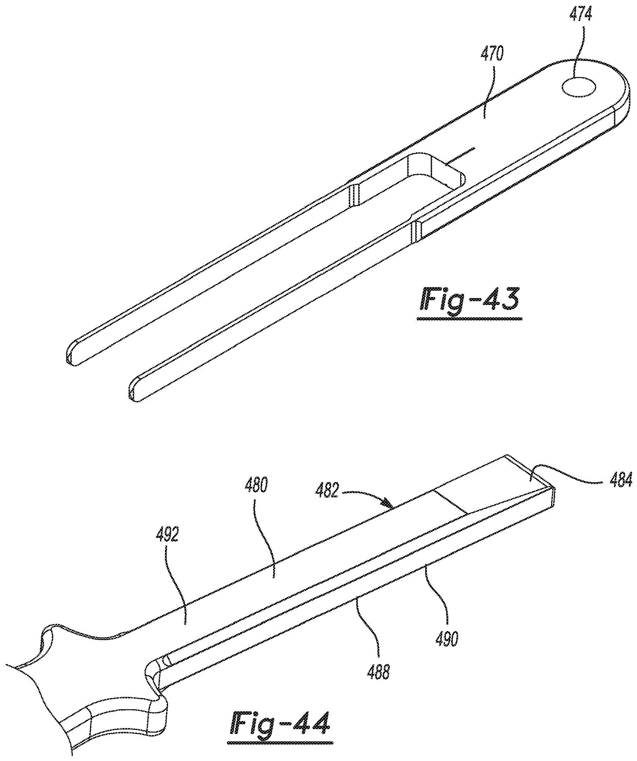

FIG. 42 illustrates a presetter tool 460. The presetter tool 460 can be used to lock tibial bearings onto a tibial tray. The presetter tool 460 comprises an arm 462 having a thin thickness that reduces potential impingement with soft tissue. FIG. 43 illustrates an alignment guide 470. The alignment guide 470 can be used to aid in the positioning of the vertical cut guide 72 (FIG. 10). The alignment guide 470 defines an aperture 474 for receipt of an alignment rod.

FIG. 44 illustrates a rasp 480. The rasp 480 includes a distal portion 482 that acts as a lead in surface that has no teeth. Such a configuration assists in avoiding the femoral condyle. The distal portion 482 has a square distal end 484 that can enable rasping of a posterior bony island. The rasp 480 can have coarse rasp teeth on sides 488 and a bottom surface 490. An upper surface 492 can have fine rasp teeth.

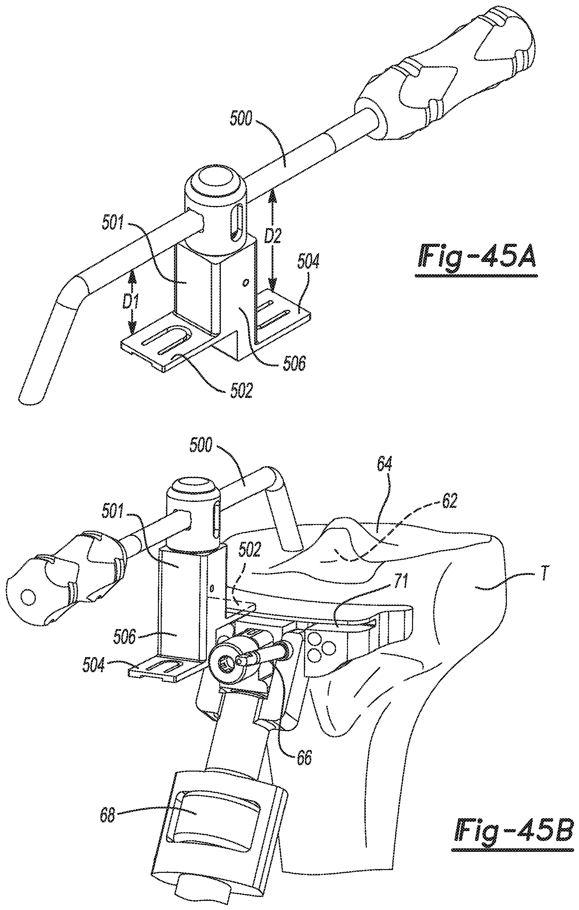

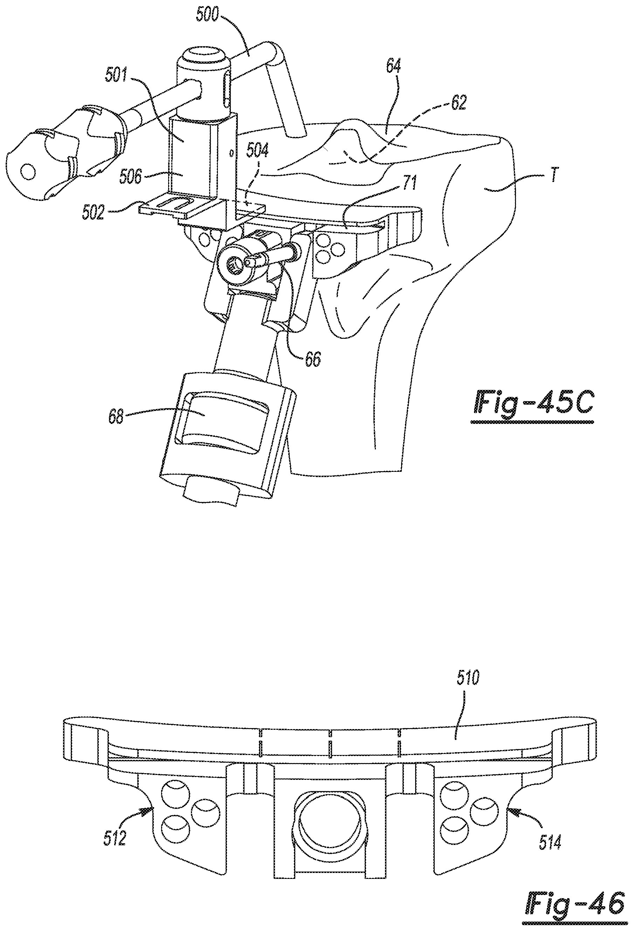

A stylus 500 constructed in accordance to one example of the present disclosure is shown in FIGS. 45A-45C. The stylus 500 can cooperate with a stylus mounting structure 501. The stylus mounting structure 501 can have a first block attachment feature 502 and a second block attachment feature 504 that oppositely extend from an attachment body 506. The first block attachment feature 502 can extend a distance D1 from the stylus 500. The second block attachment feature 504 can extend a distance D2 from the stylus 500. The distance D2 is greater than the distance D1. The first and second block attachment features 502 and 504 can both comprise a lateral projection dimensioned for receipt into the slot 71. The surgeon can select the first or second block attachment feature 502 or 504 for mating with the slot 71 (FIG. 8) depending on the more suitable distance D1 or D2 needed. As can be appreciated, the stylus 500 can be rotated 180 degrees relative to the stylus mounting structure 501 when the second block attachment feature 504 is received by the slot 71 (FIG. 8). FIG. 45B illustrates the first block attachment feature 502 mated with the slot 71. FIG. 45C illustrates the second block attachment feature 504 mated with the slot 71.

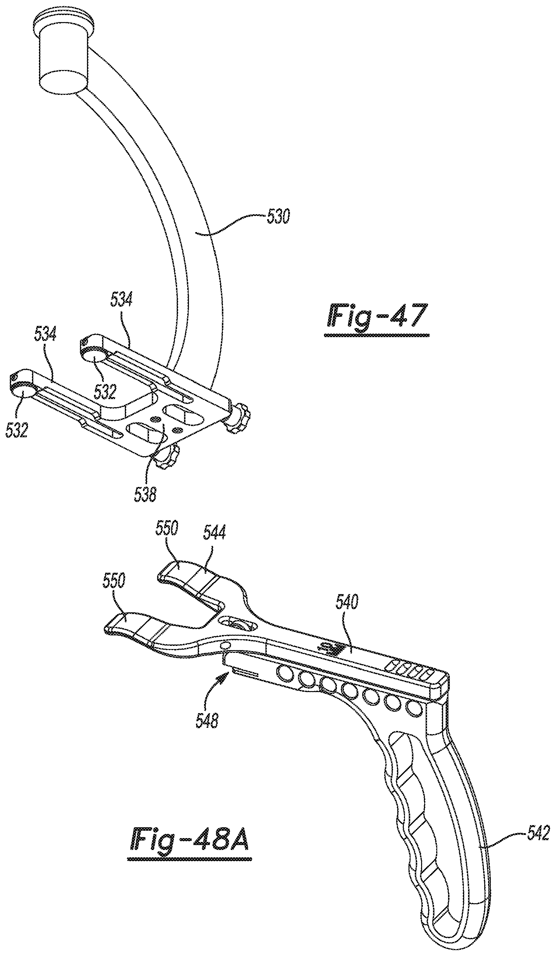

FIG. 46 illustrates a tibial resection cut block 510. The tibial resection cut block 510 has ends 512 and 514 that sweep inwardly to present a low profile as compared to the tibial resection cut block 70 (FIG. 8). The tibial resection cut block 5 10 provides a seven degree posterior slope cut inclination. FIG. 47 illustrates an impactor 530. The impactor 530 has a tray engaging portion 532 having legs 534. The legs 534 have plastic engaging pads 538 incorporated thereon.

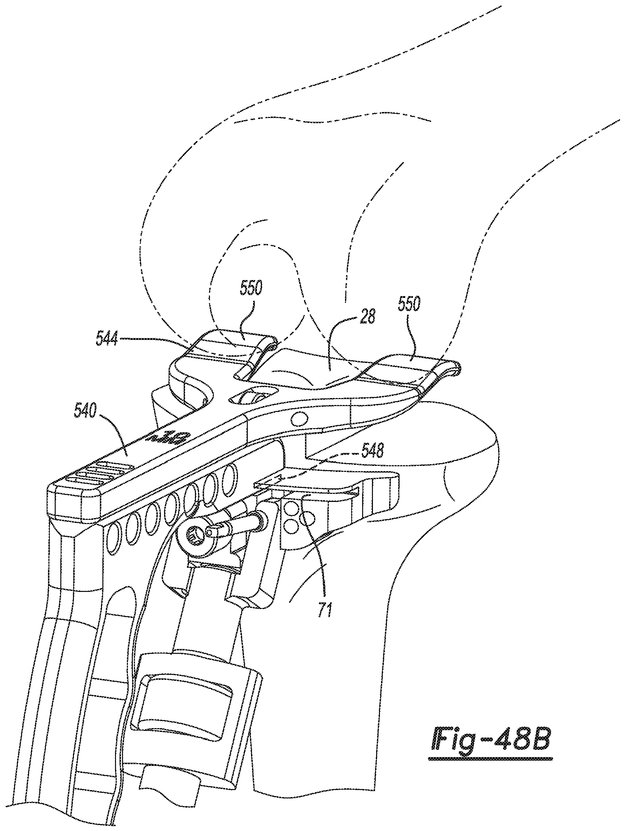

FIGS. 48A and 48B. Illustrates a tibial resection level guide 540. The tibial resection level guide 540 can include a handle end 542 and an engaging end 544. The tibial resection level guide 540 can be used to assist in determining gap tension and depth of resection. In this regard, the engaging end 544 can be inserted around the ACL island 20 to help achieve an appropriate extension/flexion space. The tibial resection level guide 540 can have an attachment 548 that can be used to attach onto the horizontal slot of the tibial cutting block (see for example slot 71 of tibial cutting block 70, FIG. 8). The engaging end 544 can include paddles 550 that are configured to engage distal femoral resection surface. In one configuration, the tibial resection level guide 540 can space tibial cutting block 19mm from the distal cut.

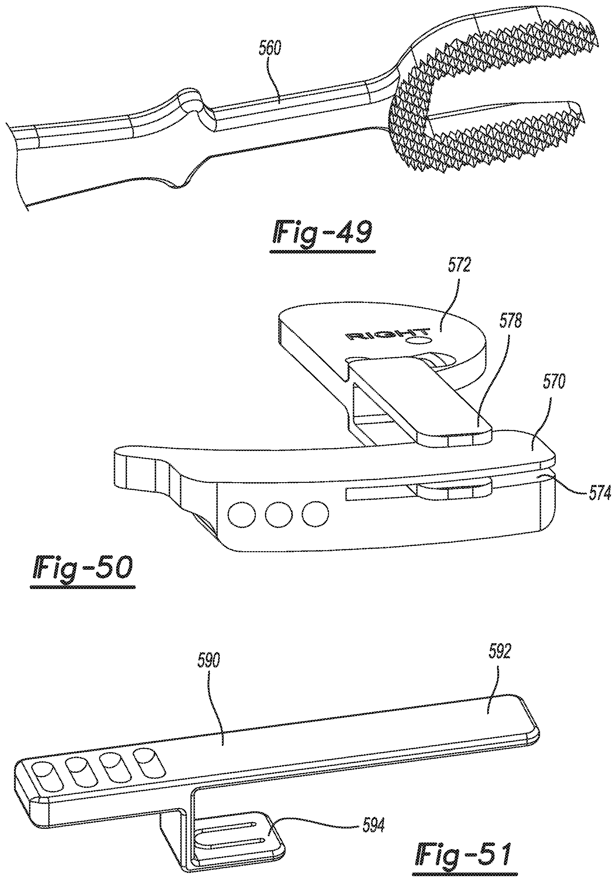

FIG. 49 illustrates a double rasp tool 560. The double rasp tool 560 can be used to rasp the medial and lateral side of the tibia concurrently. FIG. 50 illustrates a tibial resection block 570 that cooperates with a medial plate 572. The tibial resection block 570 defines a slot 574. The medial plate 572 includes a fork 578. In one method of use, if a surgeon has made a medial cut but has yet to make a lateral cut, the medial plate 572 can be laid onto the medial resection surface. The block 570 can be located relative to the medial plate 572 and be pinned to the tibia. In this regard, the block 570 may be slid medial/lateral while the fork 578 is guided along slot 574. Lateral resection can then be prepared referencing the medial resection. By directly referencing the already prepared medial cut, the lateral cut can be made more accurately. Because the fork 578 locates relative to the slot 574, the block 570 can be rotated against the tibia but the cutting plane cannot be changed. Explained further, the cutting plane realized by the already prepared medial cut will be matched with the lateral cut.

FIG. 51 illustrates an indicator tool 590. The indicator tool 590 can include an arm 592 and a finger 594. The finger 594 can locate into the slot of the tibial cutting block (see slot 71, FIG. 8). The arm 592 can be positioned to lie above the uncut tibia such that a surgeon can compare a posterior slope of the cutting block to the native posterior slope of the tibia. Before a surgeon makes any tibial cuts, the tibial cutting block (70, FIG. 8; 510, FIG. 46) can be adjusted so that the slope matches the native slope. The indicator tool 500 can be a visual aid so a surgeon can visualize the cutting block slope relative to the native slope.

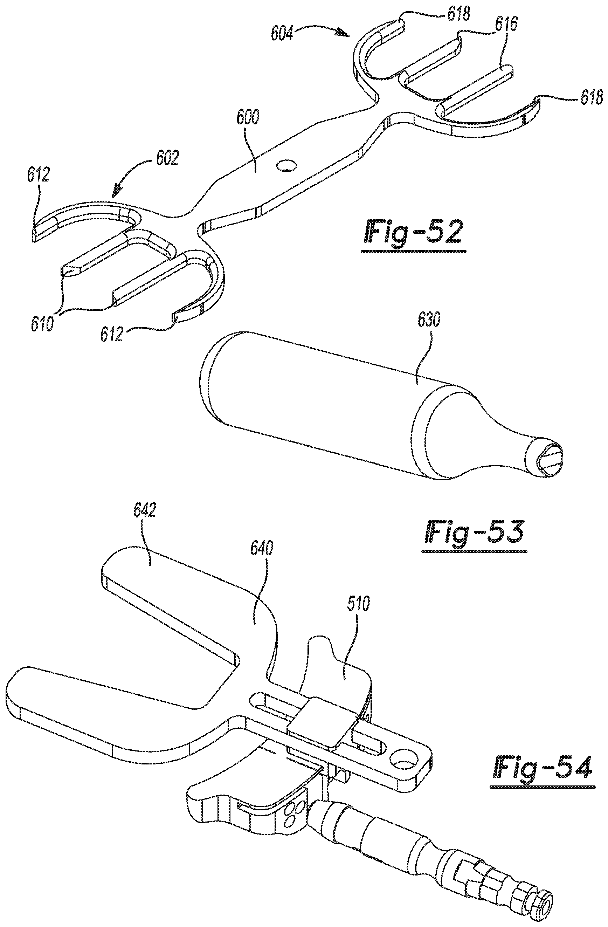

FIG. 52 illustrates a tibial template tool 600. The tibial template tool 600 can have a first template end 602 and a second template end 604. The first template end 602 can have first inner fingers 610 and first outer fingers 612. The second template end 604 can have second inner fingers 616 and second outer fingers 618. The first inner fingers 610 are spaced a first distance corresponding to a first tibial island. The second inner fingers are spaced a second distance corresponding to a second tibial island. The tibial template tool 600 can be used to accurately position the vertical cut guide 72 (FIG. 10) for creating the tibial island 20 (FIG. 4). The position and rotation of the tibial tray is determined by the position of the tibial island 20. The first outer fingers 612 can define a first outer tray footprint. The second outer fingers 618 can define a second outer tray footprint. A surgeon would benefit from knowing the ultimate position of the tray before cutting the tibia to create the tibial island 20.

The first template end 602 (or second template end 604) of the tibial template tool 600 can be laid on top of an uncut tibia T (see for example FIG. 7). The inner fingers 610 represent the bony island. The ACL will locate between the inner fingers 610. A surgeon can observe the medial/lateral coverage and tray rotation and reference either the first or second template end 602 or 604 having different sizes to represent different trays. Once the tray size and location has been determined, a marking pen can then be used to mark two lines along the inner fingers 610 to mark the preferred location of the tibial island 20. When a surgeon is ready to use the vertical cut guide 72 (FIG. 10), the vertical cut guide 72 can be positioned referencing the markings.

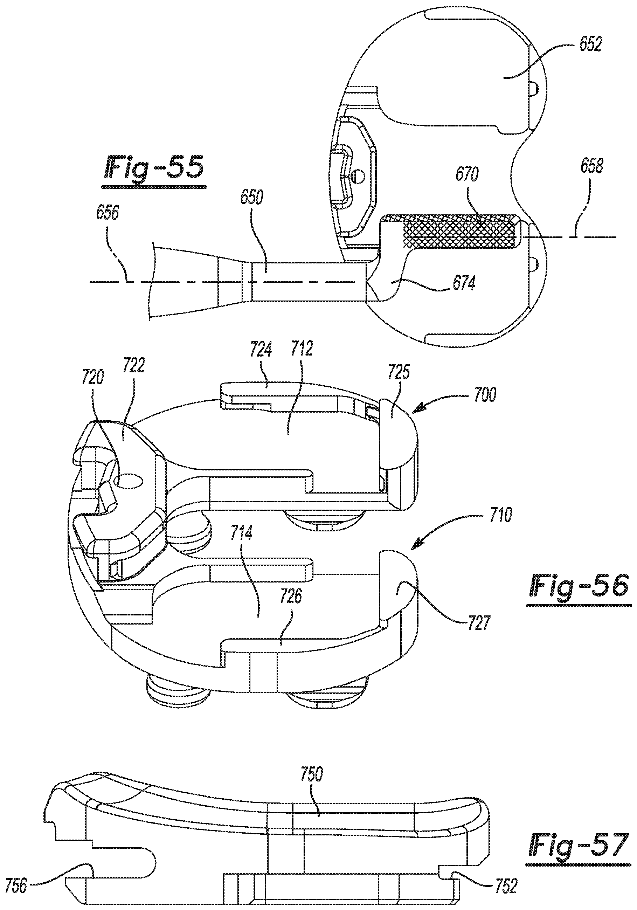

FIG. 53 illustrates a holding tool 630. The holding tool 630 can be used to hold down the bearing while the locking bar is being located. FIG. 54 illustrates a tibial recutting guide 640. The tibial recutting guide 640 can be used when an initial tibial cut was not deep enough. The tibial recutting guide 640 can include a pad member 642 that can be located onto the cut tibia. The cut block 510 can be dropped 2mm and be pinned to the tibia and the tibia can be recut. FIG. 55 illustrates an offset rasp tool 650. The offset rasp tool 650 can be used to rasp the tibial island 20 after a tray 652 has already been implanted. The offset rasp tool 650 can have a handle end 654 that extends along a first longitudinal axis 656 and a rasp end 670 that extends along a second longitudinal axis 658. The handle end 654 and the rasp end 670 are connected by an intermediate body portion 674. The first and second longitudinal axes 656 and 568 are offset allowing a surgeon to easily gain access to the tibial island 20. The rasp end 670 is dual sided allowing the surgeon to flip the tool and use it for either the medial or lateral side of the tibial island 20.

FIG. 56 illustrates a tibial tray 700 configured for implanting onto a corresponding prepared proximal tibia. The tibial tray 700, as with those described above, can be generally U-shaped and provides a slot 710 that can be configured to accommodate and provide clearance for a host ACL and/or PCL or a reconstructed ACL and/or PCL. The tray 700 can include a medial portion 712, a lateral portion 714, an anterior engagement bridge 720 and an anterior connecting portion 722. The medial portion 712 includes superiorly extending rails 724 and a posterior catch 725. The lateral portion 714 includes superiorly extending rails 726 and a posterior catch 727.

FIG. 57 illustrates a lateral bearing 750. The lateral bearing 750 can selectively secure to the lateral portion 714 of the tibial tray 700. The lateral bearing 750 includes a posterior slot 752 that is configured to locate under the posterior catch 727. The lateral bearing 750 further includes an anterior slot 756. The anterior slot 756 can align relative to the anterior connecting portion 722 for receipt of a locking bar (not shown).

The foregoing description of the examples has been provided for purposes of illustration and description. It is not intended to be exhaustive or to limit the disclosure. Individual elements or features of a particular example are generally not limited to that particular example, but, where applicable, are interchangeable and can be used in a selected example, even if not specifically shown or described. The same may also be varied in many ways. Such variations are not to be regarded as a departure from the disclosure, and all such modifications are intended to be included within the scope of the disclosure.

## Claims

1. An instrument set for preparing a proximal tibia during a bi-cruciate retaining procedure, the instrument set comprising:
a tibial resection block (54, 70) configured to be fixed to an anterior portion of the proximal tibia, the tibial resection block defining a slot (71) that extends in a medial-lateral direction when the tibial resection block is fixed to the proximal tibia; and
a stylus (60, 500) having a first block attachment feature (502, 504) that is offset from the stylus a first distance, wherein the first block attachment feature of the stylus is selectively received by the slot of the tibial resection block to position the stylus at an offset location relative to the slot;
a vertical cut guide (72) having a body, a medial arm (75c) and a lateral arm (77c) wherein a medial cut slot (74) is defined between the body and the medial arm and a lateral cut slot (76) is defined between the body and the lateral arm, the cut guide further comprising a tongue (72a) extending therefrom, the tongue configured to be received by and slidably translate along the slot of the tibial resection block;
a tibial resection level guide (540) having a handle end, an attachment portion and an engaging end, wherein the attachment portion is configured to be selectively received by the slot of the tibial resection block to selectively position the engaging end against a distal femoral resection surface; and
a tibial template tool (600) having a first template end and a second template end, the first template end having first inner fingers and first outer fingers, the second template end having second inner fingers and second outer fingers, wherein the first inner fingers are spaced a first distance corresponding to a first tibial island and the second inner fingers are spaced a second distance corresponding to a second tibial island;
wherein all of the stylus, the vertical cut guide and the tibial resection level guide are selectively and alternatively received by the slot of the tibial resection block.

2. The instrument set of claim 1 wherein the stylus further comprises a second block attachment feature, the second block attachment feature (502, 504) being offset from the stylus a second distance, wherein the first and second block attachment features of the stylus are selectively and alternatively received by the slot of the tibial resection block to position the stylus at distinct offset locations relative to the slot.

3. The instrument set of claim 2 wherein the first and second attachment features both comprise a lateral projection configured for receipt by the tibial resection block.

4. The instrument set of claim 3 wherein the first and second block attachment features oppositely extend from an attachment body.

5. The instrument set of claim 2 wherein the vertical cut guide further comprises a locking arm (72b) coupled to the cut guide and movable between an unlocked position wherein the cut guide is permitted to translate relative to the tibial resection block and a locked position wherein the locking arm engages the tibial resection block and inhibits movement of the cut guide relative to the tibial resection block.

6. The instrument set of claim 1 wherein the first outer fingers (612) define a first outer tibial tray footprint and the second outer fingers (618) define a second outer tibial tray footprint.

## Patentansprüche

1. Instrumentensatz zur Herstellung einer proximalen Tibia während eines Erhaltungsverfahrens beider Kreuzbänder, wobei der Instrumentensatz Folgendes umfasst:
einen tibialen Resektionsblock (54, 70), der dazu konfiguriert ist, an einem vorderen Abschnitt der proximalen Tibia befestigt zu sein, wobei der tibiale Resektionsblock einen Spalt (71) definiert, der sich in einer medial-lateralen Richtung erstreckt, wenn der tibiale Resektionsblock an der proximalen Tibia befestigt ist;
und einen Taststift (60, 500), der ein erstes Blockanbringungsmerkmal (502, 504) aufweist, das gegenüber dem Taststift um einen ersten Abstand versetzt ist, wobei das erste Blockanbringungsmerkmal des Taststifts selektiv von dem Spalt des tibialen Resektionsblocks aufgenommen wird, um den Taststift an einer versetzten Stelle in Bezug auf den Spalt zu positionieren;
eine vertikale Schnittführungseinrichtung (72), die einen Körper, einen medialen Arm (75c) und einen lateralen Arm (77c) aufweist, wobei ein medialer Schnittspalt (74) zwischen dem Körper und dem medialen Arm definiert ist und ein lateraler Schnittspalt (76) zwischen dem Körper und dem lateralen Arm definiert ist, wobei die Schnittführungseinrichtung ferner eine Zunge (72a) aufweist, die sich davon erstreckt, wobei die Zunge dazu konfiguriert ist, von dem Spalt des tibialen Resektionsblocks aufgenommen zu werden und daran entlang gleitend lateral bewegt zu werden;
eine Niveauausgleichseinrichtung (540) zur tibialen Resektion, die ein Griffende, einen Anbringungsabschnitt und ein Eingriffsende aufweist, wobei der Anbringungsabschnitt dazu konfiguriert ist, selektiv in dem Spalt des tibialen Resektionsblocks aufgenommen zu werden, um das Eingriffsende selektiv an einer distalen femoralen Resektionsfläche zu positionieren;
und ein Tibiaschablonenwerkzeug (600), das ein erstes Schablonenende und ein zweites Schablonenende aufweist, wobei das erste Schablonenende erste innere Finger und erste äußere Finger aufweist, wobei das zweite Schablonenende zweite innere Finger und zweite äußere Finger aufweist, wobei die ersten inneren Finger in einem ersten Abstand beabstandet sind, der einer ersten Tibiainsel entspricht und die zweiten inneren Finger in einem zweiten Abstand beabstandet sind, der einer zweiten Tibiainsel entspricht;
wobei sowohl der Taststift, als auch die vertikale Schnittführungseinrichtung, als auch die Niveauausgleichseinrichtung zur tibialen Resektion selektiv und abwechselnd von dem Spalt des tibialen Resektionsblocks aufgenommen werden.

2. Instrumentensatz nach Anspruch 1, wobei der Taststift ferner ein zweites Blockanbringungsmerkmal umfasst, wobei das zweite Blockanbringungsmerkmal (502, 504) gegenüber dem Taststift um einen zweiten Abstand versetzt ist, wobei das erste und das zweite Blockanbringungsmerkmal des Taststifts selektiv und abwechselnd von dem Spalt des tibialen Resektionsblocks aufgenommen werden, um den Taststift an verschiedenen versetzten Stellen in Bezug auf den Spalt zu positionieren.

3. Instrumentensatz nach Anspruch 2, wobei das erste und das zweite Blockanbringungsmerkmal je einen lateralen Vorsprung umfassen, der zur Aufnahme durch den tibialen Resektionsblock konfiguriert ist.

4. Instrumentensatz nach Anspruch 3, wobei das erste und das zweite Blockanbringungsmerkmal sich gegenüberliegend von einem Anbringungskörper erstrecken.

5. Instrumentensatz nach Anspruch 2, wobei die vertikale Schnittführungseinrichtung ferner einen Verriegelungsarm (72b) umfasst, der an die Schnittführungseinrichtung gekoppelt und zwischen einer nichtverriegelten Position, wobei es möglich ist, die Schnittführungseinrichtung in Bezug auf den tibialen Resektionsblock zu bewegen, und einer verriegelten Position, wobei der Verriegelungsarm den tibialen Resektionsblock in Eingriff nimmt und eine Bewegung der Schnittführungseinrichtung in Bezug auf den tibialen Resektionsblock verhindert, beweglich ist.

6. Instrumentensatz nach Anspruch 1, wobei die ersten äußeren Finger (612) einen ersten äußeren Umriss einer Tibiaplatte definieren und die zweiten äußeren Finger (618) einen zweiten äußeren Umriss einer Tibiaplatte definieren.

## Revendications

1. Ensemble d'instruments destiné à préparer un tibia proximal pendant une procédure de conservation de ligaments croisés, l'ensemble d'instruments comprenant :
un bloc de résection tibiale (54, 70) configuré pour être fixé à une partie antérieure du tibia proximal, le bloc de résection tibiale définissant une fente (71) qui s'étend dans une direction médiale-latérale quand le bloc de résection tibiale est fixé au tibia proximal ;
et un stylet (60, 500) ayant un premier élément de fixation au bloc (502, 504) qui est décalé du stylet d'une première distance, dans lequel le premier élément de fixation au bloc du stylet est reçu sélectivement par la fente du bloc de résection tibiale pour positionner le stylet au niveau d'un emplacement décalé par rapport à la fente ;
un guide de découpe vertical (72) ayant un corps, un bras médial (75c) et un bras latéral (77c) dans lequel une fente de découpe médiale (74) est définie entre le corps et le bras médial et une fente de découpe latérale (76) est définie entre le corps et le bras latéral, le guide de découpe comprenant en outre une languette (72a) s'étendant depuis celui-ci, la languette configurée pour être reçue par et effectuer une translation de manière coulissante le long de la fente du bloc de résection tibiale ;
un guide de niveau de résection tibiale (540) ayant une extrémité de poignée, une partie de fixation et une extrémité d'entrée en prise, dans lequel la partie de fixation est configurée pour être reçue sélectivement par la fente du bloc de résection tibiale pour positionner sélectivement l'extrémité d'entrée en prise contre une surface de résection fémorale distale ;
et un outil de gabarit tibial (600) ayant une première extrémité de gabarit et une deuxième extrémité de gabarit, la première extrémité de gabarit ayant des premiers doigts internes et des premiers doigts externes, la deuxième extrémité de gabarit ayant des deuxièmes doigts internes et des deuxièmes doigts externes, dans lequel les premiers doigts internes sont espacés d'une première distance correspondant à un premier îlot tibial et les deuxièmes doigts internes sont espacés d'une deuxième distance correspondant à un deuxième îlot tibial ;
dans lequel le stylet, le guide de découpe vertical et le guide de niveau de résection tibiale sont tous reçus sélectivement et alternativement par la fente du bloc de résection tibiale.

2. Ensemble d'instruments selon la revendication 1 dans lequel le stylet comprend en outre un deuxième élément de fixation au bloc, le deuxième élément de fixation au bloc (502, 504) étant décalé du stylet d'une deuxième distance, dans lequel les premier et deuxième éléments de fixation au bloc du stylet sont reçus sélectivement et alternativement par la fente du bloc de résection tibiale pour positionner le stylet au niveau d'emplacements décalés distincts par rapport à la fente.

3. Ensemble d'instruments selon la revendication 2 dans lequel les premier et deuxième éléments de fixation comprennent tous les deux une saillie latérale configurée pour être reçue par le bloc de résection tibiale.

4. Ensemble d'instruments selon la revendication 3 dans lequel les premier et deuxième éléments de fixation au bloc s'étendent en opposition depuis un corps de fixation.

5. Ensemble d'instruments selon la revendication 2 dans lequel le guide de découpe comprend en outre un bras de verrouillage (72b) couplé au guide de découpe et capable de mouvement entre une position déverrouillée dans laquelle il est permis au guide de découpe d'effectuer une translation par rapport au bloc de résection tibiale et une position verrouillée dans laquelle le bras de verrouillage entre en prise avec le bloc de résection tibiale et inhibe le mouvement du guide de découpe par rapport au bloc de résection tibiale.

6. Ensemble d'instruments selon la revendication 1 dans lequel les premiers doigts externes (612) définissent une première empreinte de plateau tibial externe et les deuxièmes doigts externes (618) définissent une deuxième empreinte de plateau tibial externe.
